(19) Europäisches Patentamt
European Patent Office
Office européen des brevets

(11) Veröffentlichungsnummer: **0 102 559**
**B1**

(12) # EUROPÄISCHE PATENTSCHRIFT

(45) Veröffentlichungstag der Patentschrift: **07.01.87**

(51) Int. Cl.⁴: **C 07 D 249/08**, A 01 N 43/653

(21) Anmeldenummer: **83107939.7**

(22) Anmeldetag: **11.08.83**

(54) **Neopentyl-phenethyltriazole, Verfahren zu ihrer Herstellung und diese enthaltende Fungizide.**

(30) Priorität: **07.09.82 DE 3233145**

(43) Veröffentlichungstag der Anmeldung: **14.03.84 Patentblatt 84/11**

(45) Bekanntmachung des Hinweises auf die Patenterteilung: **07.01.87 Patentblatt 87/2**

(84) Benannte Vertragsstaaten: **AT BE CH DE FR GB IT LI NL SE**

(56) Entgegenhaltungen:
**DE - A - 2 610 022**
**DE - A - 2 654 890**
**GB - A - 1 533 706**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Buschmann, Ernst, Dr., Georg-Ludwig-Krebs-Strasse 10, D-6700 Ludwigshafen (DE)**
Erfinder: **Schulz, Guenter, Dr., An der Froschlache 3, D-6700 Ludwigshafen (DE)**
Erfinder: **Heilen, Gerd, Dr., Schifferstadter Strasse 1b, D-6720 Speyer (DE)**
Erfinder: **Pommer, Ernst-Heinrich, Dr., Berliner Platz 7, D-6703 Limburgerhof (DE)**
Erfinder: **Ammermann, Eberhard, Dr., Sachsenstrasse 3, D-6700 Ludwigshafen (DE)**

EP 0 102 559 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft alpha-Neopentyl-phenethyltriazole und ihre Salze und Metallkomplexe sowie fungizide Mittel, die diese Verbindungen enthalten und Verfahren zu ihrer Herstellung.

Triazolderivate z.B. Verbindung A werden in DE-OS 27 39 352 beschrieben.

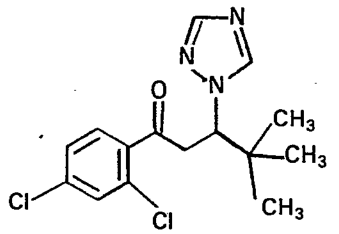

A

Verbindungen dieses Typs zeigen im wesentlichen pflanzenwuchshemmende Wirkung.

Es ist ferner bekannt, die Verbindung 1-(2,4-Dichlorphenyl)-3-(1,2,4-triazolyl-1)-4,4-dimethylpentanol-1 (B) zur Regulierung des Pflanzenwachstums zu verwenden (DE-OS 27 38 725).

Es ist ferner bekannt, die Verbindung 1-(2,4-Dichlorphenyl)-3- (1,2,4-triazolyl-1)- 4,4-dimethylpentan-1-on-oxim (C) zur Regulierung des Pflanzenwachstums zu verwenden (DE-OS 28 42 801).

Es wurde nun gefunden, dass alpha-Neopentyl-phenethyltriazole der Formel I

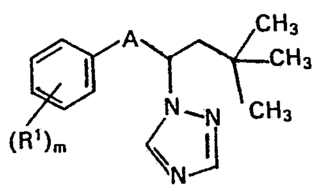

(I),

in der $R^1$ $C_1$–$C_4$-Alkyl, Phenyl, $CF_3$, $C_1$–$C_4$-Alkoxy oder Halogen,

m, 0, 1, 2, 3,

A C = X, CHY oder $CR^2R^3$ bedeuten, wobei X für 0 und $NOR^4$ und Y für $OR^5$, $OC_1$–$C_4$ -Acyl, OBenzoyl oder $OSi(CH_3)_3$ stehen und $R^2$ OH oder $OCH_3$,

$R^3$ $C_1$–$C_4$-Alkyl, Benzyl, Phenyl, 4-Cl-Benzyl, 4-$CH_3$-Phenyl, 4-Cl-Phenyl, 4-Br-Phenyl oder Vinyl, $R^4$ und $R^5$ H, $C_1$–$C_6$-Alkyl, Allyl, Propargyl, Benzyl, 4-F-Benzyl oder 4-$CH_3$-Benzyl bedeuten, sowie die pflanzenverträglichen Salze und Metallkomplexe dieser Verbindungen eine gute fungizide Wirkung haben, die der Wirkung der bekannten Triazolderivate überlegen ist.

$R^1$ bedeutet beispielsweise Methyl, Ethyl, n-Propyl, i-Propyl, n-Butyl, tert.-Butyl, Phenyl, $CF_3$, Methoxy, Ethoxy, Isopropoxy, Fluor, Chlor, Brom, Jod.

$R^3$ bedeutet beispielsweise Methyl, Ethyl, n-Propyl, n-Butyl, Phenyl, 4-$CH_3$-Phenyl, 4-Cl-Phenyl, 4-Br-Phenyl, Vinyl, Benzyl, 4-Cl-Benzyl.

$R^4$ und $R^5$ stehen z.B. für Wasserstoff, Methyl, Ethyl, Propyl, i-Propyl, Butyl, i-Butyl, Pentyl, 3,3-Dimethylbutyl, Hexyl, Benzyl, 4-F-Benzyl, 4-$CH_3$-Benzyl, Allyl, Propargyl.

Pflanzenphysiologisch verträgliche Salze der Verbindungen sind z.B. Hydrochloride, Hydrobromide, Sulfate, Phosphate, Oxalate, Acetate, Formiate, Nitrate oder Dodecylbenzolsulfonate. Metallkomplexe sind z.B. die Komplexe mit Kupfer oder Zink.

Die neuen Verbindungen enthalten ein oder auch zwei asymmetrische C-Atome. Sie kommen daher in Form ihrer optischen Isomeren vor. Die Erfindung umfasst sowohl die reinen optischen Isomeren als auch ihre Mischungen.

Schema 1 zeigt auf, wie die erfindungsgemässen Verbindungen mit allgemein bekannten Reaktionen hergestellt werden können.

Schema 1:

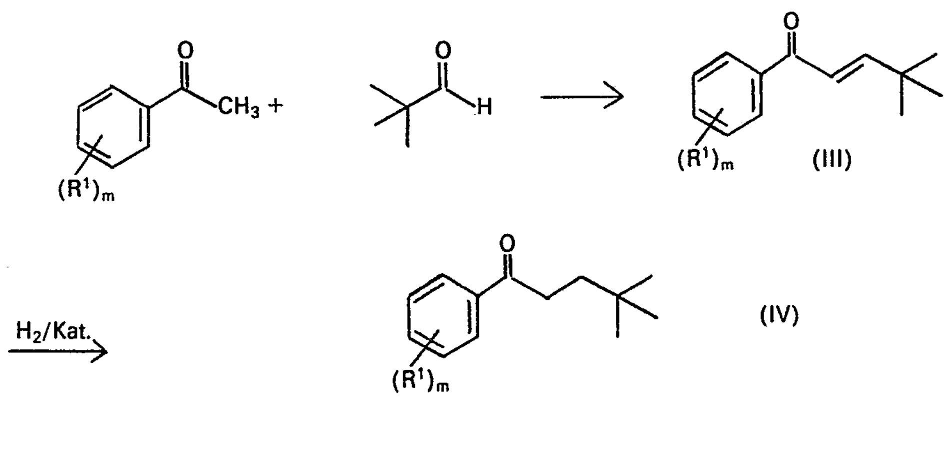


(III)

(IV)

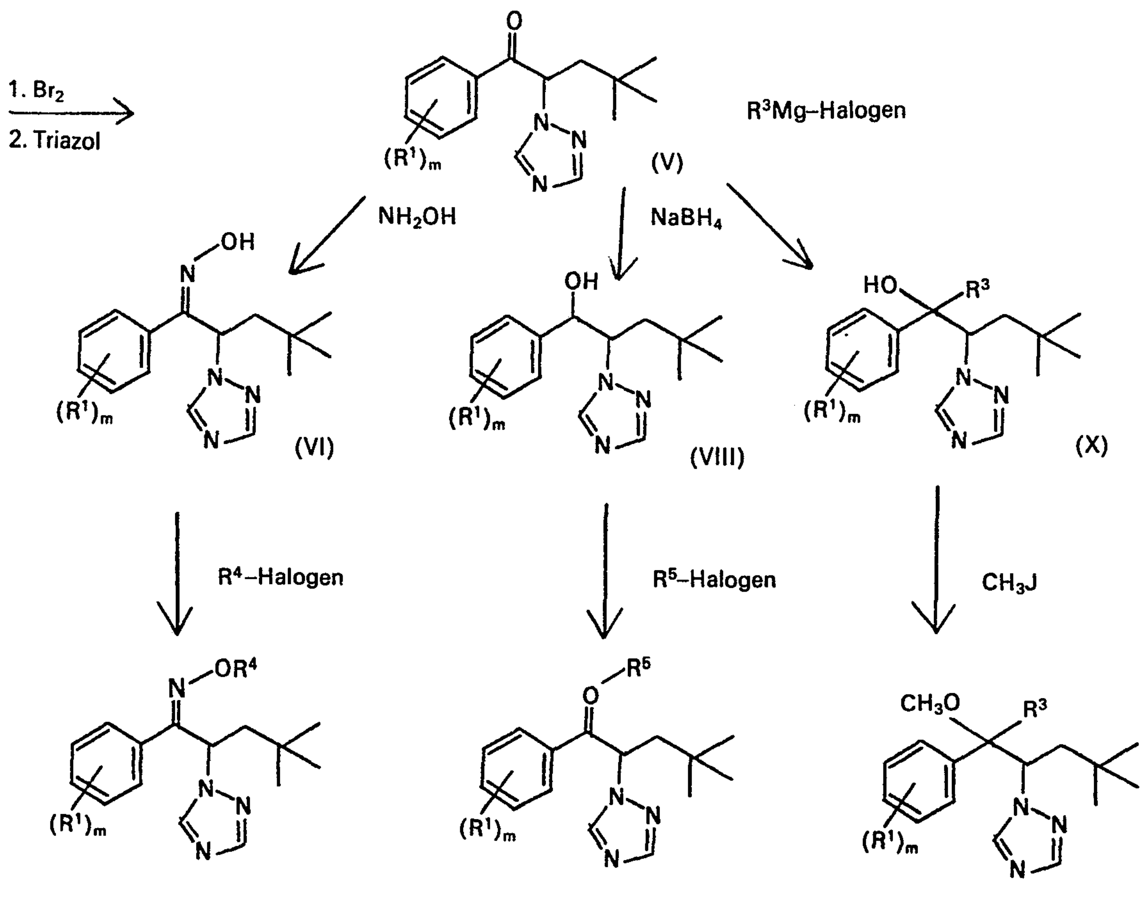


(V) (VI) (VIII) (X) (VII) (IX) (XI)

Es erweist sich oft als zweckmässig, den Triazolring in einer späteren Synthesephase einzuführen wie z.B. Schema 2 zeigt:

Schema 2:

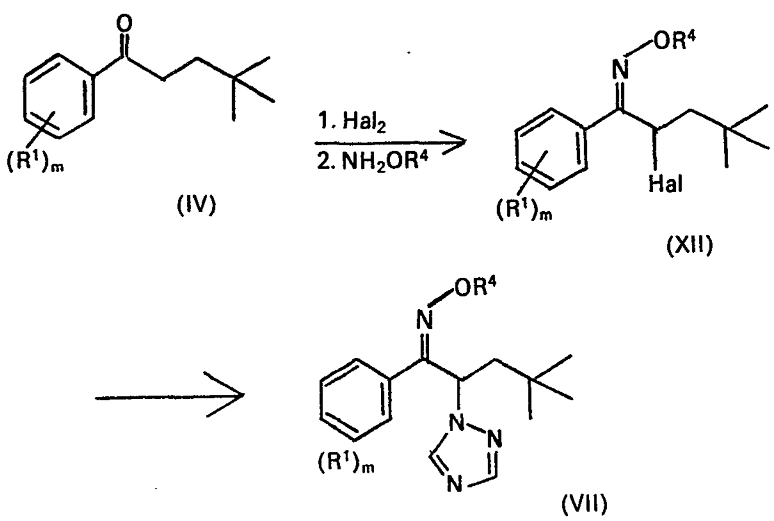


(IV) (XII) (VII)

Hal = Halogen

Das Verfahren gemäss Beispiel 1, 2 und 3 wird bevorzugt.

Die folgenden Vorschriften und Beispiele schildern die Herstellung der erfindungsgemässen Substanzen:

Vorschrift 1

Herstellung der α,β-ungesättigten Ketone (Formel III, Schema 1):

z.B.

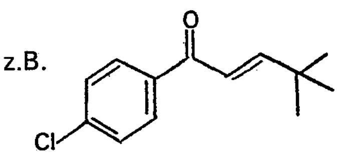

(IIIa)

Zu einer Lösung von 773 g 4-Chloracetophenon und 25 g NaOH in 1400 ml Ethanol wurden innerhalb von 4 Stunden 430 g Pivalaldehyd in 700 ml Ethanol bei Raumtemperatur (20 °C) zugetropft. Man rührte 1 Stunde nach, neutralisierte mit Eisessig und engte ein. Der Rückstand wurde mit $CH_2Cl_2/H_2O$ aufgenommen. Die organische Phase wurde mit Wasser gewaschen, über $Na_2SO_4$ getrocknet und eingeengt. Destillation des Rückstandes ergab 520 g der Verbindung IIIa, Sdp. 117–121 °C/0,2 mbar.

Vorschrift 2

Herstellung der Dimethylbutyrylbenzole (Formel IV, Schema 1, 2):

z.B.

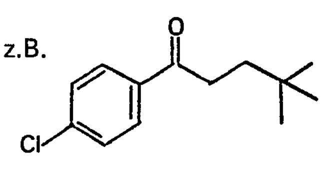

(IVa)

200 g IIIa werden in 500 ml Methanol gelöst. Nach Zugabe von 20 g Hydrierkontakt (5% Pr, 0,5% Pd auf $Al_2O_3$) wird bei 130 °C und 20 bar hydriert bis die Wasserstoffaufnahme beendet ist. Der Kontakt wird abfiltriert. Das Filtrat wird eingeengt. Man destilliert den Rückstand im Vakuum. Ausbeute 165 g der Verbindung IVa, Sdp. 106–108 °C, 0,1 mbar.

Beispiel 1

Herstellung des alpha-Triazolylketons entsprechend Formel V (Schema 1 der Formel)

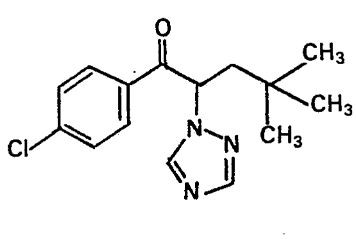

(Va)

a) Zur Lösung von 100 g der Verbindung IVa in 1 Liter Diethylether wurden bei Raumtemperatur (20 °C) 71,2 g $Br_2$ zugetropft. Man rührte 1 Stunde nach, goss auf Eiswasser, wusch die Etherphase mit Wasser, trocknete sie mit $Na_2SO_4$ und engte ein. Das so erhaltene Bromketon wurde ohne weitere Reinigung weiter wie folgt umgesetzt:

b) Zur Lösung von 162 g 1,2,4-Triazol und 249 g $Na_2CO_3$ in 1 Liter Ethanol wurde unter Rückfluss 357 g des gemäss a) erhaltenen Bromketons zugetropft. Man erwärmte 3 Stunden unter Rückfluss, engte ein, nahm mit $CH_2Cl_2/H_2O$ auf, wusch die organische Phase mit Wasser, trocknete über $Na_2SO_4$, engte ein und destillierte. Man erhielt 294 g der Verbindung Va, als gelbliches Öl, Sdp. 180–184 °C/0,4 mbar (Verbindung Nr. 1).

Durch Einleiten von HCl-Gas in eine etherische Lösung der Triazolylketone V lassen sich die Hydrochloride fällen. Tropft man zu etherischen Lösungen der Triazolylketone konz. $HNO_3$ zu, dann werden die entsprechenden Nitrate gefällt.

Wie im obigen Beispiel beschrieben, lassen sich die folgenden alpha-Triazolylketone der Formel V herstellen:

| Nr. | $R^1$ | m | Kp °C/mbar | Fp. °C Z = Zersetzung |
|---|---|---|---|---|
| 1 | 4–Cl | 1 | 180–184 0,4 | |
| 2 | 2,4–$Cl_2$ | 2 | 200–205 0,6 | |
| 3 | 4–Cl | 1 | Hydrochlorid | 130 Z |
| 4 | 2,4–$Cl_2$ | 2 | Hydrochlorid | 147 Z |
| 5 | H | 0 | | |
| 6 | 4–$CH_3$ | 1 | 166–169 0,3 | |
| 7 | 4–Br | 1 | | |
| 8 | 4–t–Bu | 1 | | |
| 9 | 4–$C_6H_5$ | 1 | | |
| 10 | 4–$CF_3$ | 1 | | |
| 11 | 4–$OCH_3$ | 1 | | |
| 12 | 2,3,4–Cl | 3 | | |

Beispiel 2

Herstellung des Ketoxims (Formel VI, Schema 1)

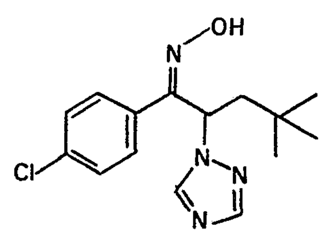

(VIa)

Zur Lösung von 37 g der Verbindung Va in 350 ml Ethanol wurden 8,6 g $NH_3^{\oplus}$ $OHCl^{\ominus}$ und 18,6 g Natriumacetat zugegeben. Nach 3 Stunden Kochen unter Rückfluss wurde eingeengt,

mit $CH_2Cl_2/H_2O$ aufgenommen, mit Wasser gewaschen, über $Na_2SO_4$ getrocknet und eingeengt. Der Rückstand kristallisierte beim Verreiben mit Ether, 14 g Ausbeute an Verbindung VIa, Fp. 136 °C (Verbindung Nr. 13).

Beispiel 3

Herstellung des Ketoximethers (Formel VII, Schema 1)

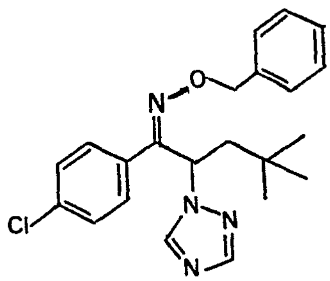

× $HNO_3$ (VIIa)

Zu einem Gemisch von 30,6 g Oxim VIa, 100 ml Toluol, 12 g NaOH, 100 ml $H_2O$ und 1 g Tetrabutylammoniumjodid wurden bei 80 °C 25,3 g Benzylchlorid gelöst in 50 ml Toluol zugetropft. Man rührte 8 Stunden bei 80 °C, wusch die organische Phase mehrfach mit Wasser, trocknete sie über $Na_2SO_4$ und engte ein. Der Rückstand wurde in Ether gelöst. Unter Eiskühlung wurden 10 g konz. $HNO_3$ zugetropft. Das auskristallisierte Nitrat VIIa wurde abgesaugt, mit Ether gewaschen und getrocknet. Fp. 112 °C (Zersetzung) (Verbindung Nr. 14).

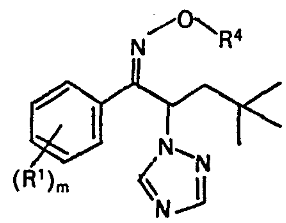

x HX

Wie im obigen Beispiel beschrieben, lassen sich die folgenden alpha-Triazolyloxime und Oximether herstellen.

| Nr. | $(R^1)_m$ | $R^4$ | Fp/ °C | HX |
|---|---|---|---|---|
| 13 | 4-Cl | H | 136 | |
| 14 | 4-Cl | $CH_2C_6H_5$ | 112 Z | $HNO_3$ |
| 15 | 4-Cl | $CH_3$ | Harz | |
| 16 | 4-Cl | $CH_3$ | 176 Z | HCl |
| 17 | 4-Cl | $CH_2CH=CH_2$ | | |
| 18 | 4-Cl | $CH_2$-4-Cl-$C_6H_4$ | | |
| 19 | 2,4-$Cl_2$ | $CH_3$ | Harz | |
| 20 | 2,4-$Cl_2$ | $CH_3$ | 148 Z | $HNO_3$ |
| 21 | 2,4-$Cl_2$ | $CH_2CH=CH_2$ | Harz | |
| 22 | 2,4-$Cl_2$ | Propargyl | Harz | |
| 23 | 2,4-$Cl_2$ | $CH_2C_6H_5$ | Harz | |
| 24 | 2,4-$Cl_2$ | $CH_2$-4-F-$C_6H_4$ | Harz | |
| 25 | 2,4-$Cl_2$ | $CH_2$-3-F-$C_6H_4$ | Harz | |
| 26 | 2,4-$Cl_2$ | $CH_2$-2-F-$C_6H_4$ | Harz | |
| 27 | 2,4-$Cl_2$ | $CH_2$-2,3,6-$Cl_3$-$C_6H_2$ | Harz | |
| 28 | 2,4-$Cl_2$ | $CH_2$-2,6-$Cl_2$-$C_6H_3$ | Harz | |
| 29 | 2,4-$Cl_2$ | $CH_2$-2J-$C_6H_4$ | 101–104 | |
| 30 | H | $CH_3$ | | |
| 31 | 4-$C_6H_5$ | $CH_3$ | 149 | |
| 32 | 2,4-$Cl_2$ | H | Harz | |
| 33 | 2,4-$Cl_2$ | $CH_2$-2-Cl-6-F-$C_6H_3$ | Harz | |

Beispiel 4

Herstellung des Triazolylcarbinols (Formel VIII, Schema 1):

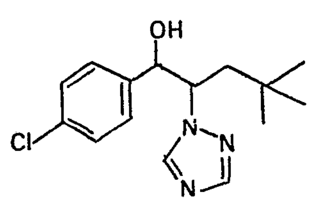

x HCl (VIIIa)

Zur Lösung von 35 g des Ketons Va in 200 ml Methanol wurden 20 g $NaBH_4$ portionsweise zugegeben. Nach einer Stunde Rückfluss wurde eingeengt, mit verd. HCl kurz aufgekocht, mit wässriger NaOH alkalisch gestellt und mit $CH_2Cl_2$ extrahiert. Die org. Phase wurde mit Wasser gewaschen, über $Na_2SO_4$ getrocknet, und eingeengt. In die Lösung des Rohproduktes in Ether wurde HCl-Gas eingeleitet. Das ausgefallene Hydrochlorid wurde abgesaugt, mit Ether gewaschen und getrocknet – farblose Kristalle, Fp. 200 bis 202 °C (Zersetzung) (Verbindung Nr. 38) Neutralisation des Hydrochlorids in methanoli-

scher $NH_3$-Lösung liefert das Carbinol VIIIb; Kp. 186–200 °C/0,4 mbar; Fp. 126–128 °C.

Wie oben beschrieben lassen sich die folgenden Triazolylalkohole der Formel VIII herstellen

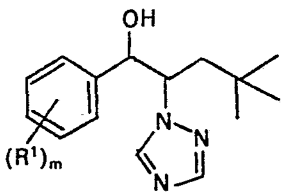

x HX

| Nr. | $(R^1)_m$ | HX | Kp | °C/mm Hg | Fp. °C |
|---|---|---|---|---|---|
| 38 | 4–Cl | HCl | | | 200–202 |
| 34 | 2,4–$Cl_2$ | | | | 160 |
| 35 | H | | | | |
| 36 | 4–$CH_3$ | | 186–192 | 0,3 n | |
| 37 | 4–Br | | | | |
| 43 | 4–Cl | | | | |
| 39 | 4–$C_6H_5$ | | | | |
| 40 | 4–$CF_3$ | | | | |
| 41 | 4–$OCH_3$ | | | | |
| 42 | 2,3,4–$Cl_3$ | | | | |

Beispiel 5

Herstellung des Triazolylethers (Formel IX, Schema 1):

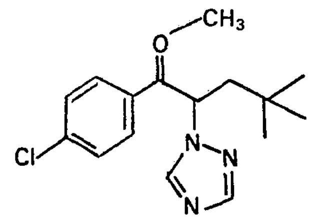

(IXa)

Zu einer Lösung von 70 g Carbinol VIIIb und 37,3 g Methyljodid in 220 ml Dimethylsulfoxid und 770 ml Diethylether wurden portionenweise 15,9 g NaH (80%, Paraffinsuspension) zugegeben. Nach Ende der $H_2$-Entwicklung wurde 1/2 Stunde unter Rückfluss erwärmt. Man liess abkühlen, versetzte mit 1 Liter Eiswasser, extrahierte mehrfach mit Ether, trocknete über $Na_2SO_4$, engte ein und destillierte. Ausbeute 50 g der Verbindung IXa, gelbliches Öl, Sdp. 140–144 °C/0,3 mbar (Verbindung Nr. 56).

Aus der n-Pentan-Lösung des Triazolylethers IXa lässt sich das Hydrochlorid fällen. Fp. 68 °C Z. (Verbindung Nr. 44).

Wie oben beschrieben lassen sich die folgenden Triazolylether der Formel IX herstellen.

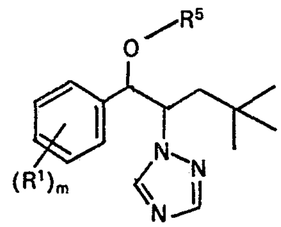

x HX (IX)

| Nr. | $(R^1)_m$ | $R^5$ | HX | Fp. °C Sdp. |
|---|---|---|---|---|
| 56 | 4–Cl | $CH_3$ | | 140–144 0,3 mbar |
| 44 | 4–Cl | $CH_3$ | HCl | 68 |
| 45 | 4–Cl | $C_2H_5$ | | 158–166 0,4 mbar |
| 46 | 4–Cl | $CH_2CH=CH_2$ | | 156–167 0,1 mbar |
| 47 | 4–Cl | Propargyl | | |
| 48 | 4–Cl | $CH_2C_6H_5$ | | |
| 49 | 2,4–$Cl_2$ | $CH_3$ | | 142–146 0,3 mbar |
| 50 | 2,4–$Cl_2$ | $CH_3$ | HCl | 194 |
| 51 | 2,4–$Cl_2$ | $C_2H_5$ | | 140–148 0,2 mbar |
| 52 | 2,4–$Cl_2$ | $C_2H_5$ | HCl | 162 |
| 53 | H | $CH_3$ | | |
| 54 | 4–Br | $CH_3$ | | |
| 55 | 4–$CF_3$ | $CH_3$ | | |

Beispiel 6

Herstellung des Triazolylcarbinols (Formel X, Schema 1):

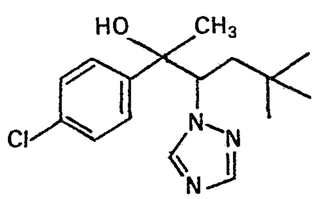

(Xa)

Zu einer Grignardverbindung, hergestellt aus 10,3 g Mg-Spänen und 61 g $CH_3J$ in 300 ml Ether wurden 50 g Triazolylketon Va in 100 ml Ether gelöst zugetropft. Man erwärmte 2 Stunden unter Rückfluss und hydrolysierte mit konzentrierter wässriger $NH_4Cl$-Lösung.

Die org. Phase wurde mehrfach mit Wasser gewaschen, über $Na_2SO_4$ getrocknet und eingeengt. Destillation des Rückstandes ergab 39 g gelbliches Harz. Sdp. 170–172 °C/0,4 mbar (Verbindung Nr. 71).

Wie oben beschrieben lassen sich die folgenden Triazolylcarbinole X herstellen.

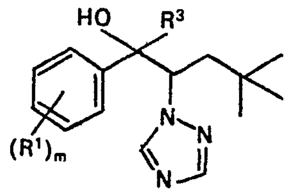

x HX (X)

| Nr. | $(R^1)_m$ | $R^3$ | HX | Fp. °C Sdp. |
|---|---|---|---|---|
| 71 | 4–Cl | $CH_3$ | | 170–172 0,4 mbar |
| 57 | 4–Cl | $CH_2C_6H_5$ | | Harz |
| 58 | 4–Cl | $CH=CH_2$ | | Harz |
| 59 | 4–Cl | $4-Cl-C_6H_4$ | | 190 |
| 60 | 4–Cl | n-Propyl | | Harz |
| 61 | 4–Cl | $C_6H_5$ | $HNO_3$ | 170 Z |
| 62 | 4–Cl | $C_2H_5$ | | Harz |
| 63 | $2,4-Cl_2$ | $CH_3$ | | |
| 64 | $2,4-Cl_2$ | $C_6H_5$ | | 176 |
| 65 | $2,4-Cl_2$ | $C_2H_5$ | | Harz |
| 66 | $2,4-Cl_2$ | n-Propyl | | Harz |
| 67 | $2,4-Cl_2$ | $CH=CH_2$ | | 166–168 0,4 mbar |
| 68 | H | $CH_3$ | | |
| 69 | 4–Br | $CH_3$ | | |
| 70 | $4-CH_3$ | $CH_3$ | | |

Beispiel 7

Herstellung des Triazolylethers (Formel XI, Schema 1):

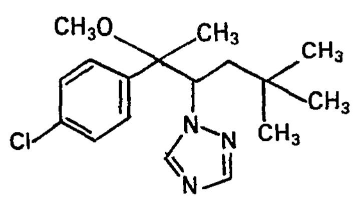

(XIa)

Zur Lösung von 30,8 g Carbinol Xa und 18,5 g Jodmethan in 390 ml Diethylether und 110 ml Dimethylsulfoxid wurden 6,2 g NaH portionsweise zugegeben. Nach Ende der $H_2$-Entwicklung wurde 1/2 Stunde unter Rückfluss erwärmt. Nach dem Abkühlen versetzte man mit 600 ml $H_2O$, extrahierte mehrfach mit Ether, trocknete über $Na_2SO_4$ und engte ein. Destillation des Rückstandes ergab 18 g gelbliches Öl, Sdp. 180–182 °C/0,4 mbar (Verbindung Nr. 77).

Wie oben beschrieben, lassen sich die folgenden Triazolylether XI herstellen.

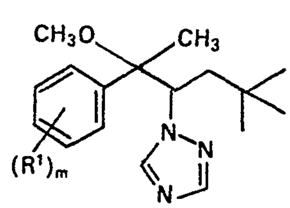

x HX (XI)

| Nr. | $(R^1)_m$ | HX | Fp. °C Sdp. |
|---|---|---|---|
| 77 | 4–Cl | | 180–182 0,4 mbar |
| 72 | 4–Cl | $HNO_3$ | |
| 73 | $2,4-Cl_2$ | | |
| 74 | H | | |
| 75 | 4–Br | | |
| 76 | $4-CH_3$ | | |

Beispiel 8

Herstellung des Metallkomplexes der Formel

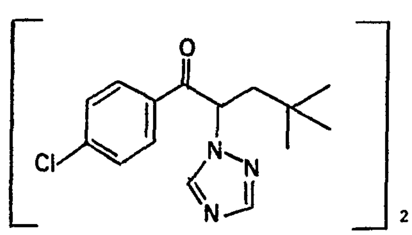

$CuCl_2$ (XII)

Man vereinte die ethanolischen Lösungen von 9 g $CuCl_2 \times 2\,H_2O$ und 23 g der Verbindung Va und erwärmte kurz unter Rückfluss. Nach dem Abkühlen gab man Wasser zu und verrieb das ausgefallene Rohprodukt mit Wasser. Das krist. Produkt wurde abgesaugt und im Vakuum getrocknet. Ausbeute 15 g, blaues Pulver Fp. 90 °C (Verbindung Nr. 78).

Beispiel 9

Herstellung des Acyloxytriazols der Formel XIIIa

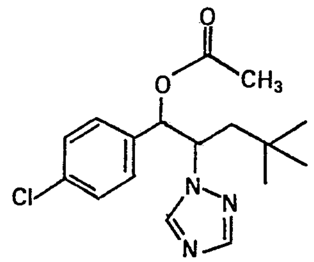

$(XIII_a)$

Zur Lösung von 127 g der Verbindung VIIIb, 51 g Pyridin und 0,2 g 4-Dimethylaminopyridin in 1 Liter Tetrahydrofuran wurden 44 g Acetanhydrid zugetropft. Man rührte 14 Stunden bei Raumtemperatur (20 °C), engte ein und nahm den Rückstand in $CH_2Cl_2/H_2O$ auf. Die organische Phase wurde mehrfach mit Wasser gewaschen, über $Na_2SO_4$ getrocknet und eingeengt. Destillation des Rückstandes, Sdp. 170–175 °C/0,1 mbar ergab 75 g XIIIa gelbliches Öl (Verbindung Nr. 84).

Wie oben beschrieben lassen sich die folgenden Acyloxyverbindungen der Formel XIII herstellen:

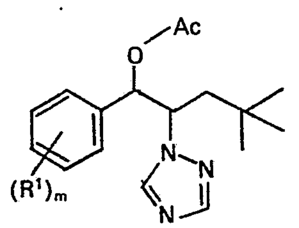

(XIII)

| Nr. | $(R^1)_m$ | Ac | Sdp. °C/mbar |
|---|---|---|---|
| 84 | 4–Cl | Acetyl | 170–175 0,1 mbar |
| 79 | 4–Cl | Propionyl | |
| 80 | 4–Cl | Benzoyl | |
| 81 | 4–Br | Acetyl | |
| 82 | 2,4–$Cl_2$ | Acetyl | |
| 83 | 4–$CH_3$ | Acetyl | |

Beispiel 10

Herstellung des Silylethers der Formel XIVa

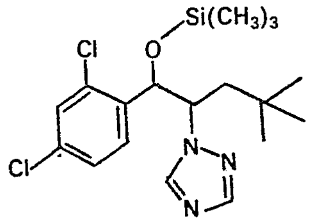

(XIVa)

20 g Triazolylalkohol (Verbindung Nr. 34) und 17 g N-Trimethylsilylacetamid wurden in 200 ml Toluol 3 Stunden unter Rückfluss erhitzt.

Die abgekühlte Lösung wurde mit Wasser gewaschen, über $Na_2SO_4$ getrocknet und eingeengt. Als Rückstand verblieben 18 g der Verbindung XIVa als farblose Kristalle. Schmp. 67 °C (Verbindung Nr. 85).

Wie oben beschrieben lassen sich die folgenden Silylether der Formel XIV herstellen.

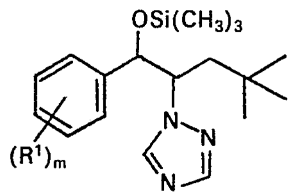

(XIV)

| Nr. | $(R^1)_m$ | Fp. °C |
|---|---|---|
| 89 | 4–Cl | |
| 85 | 2,4–$Cl_2$ | 67 |
| 86 | 4–$CH_3$ | |
| 87 | 4–Br | |
| 88 | H | |

Die erfindungsgemässen Verbindungen und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden. Ferner können sie auch im Materialschutz, u.a. zur Bekämpfung holzzerstörender Pilze, verwendet werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie Gemüse – wie Gurken, Bohnen und Kürbisgewächse. –

Die Verbindungen sind insbesondere geeignet zur Bekämpfung folgender Pflanzenkrankheiten:

Erysiphe graminis (echter Mehltau) in Getreide,
Erysiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Phythophthora infestans (falscher Mehltau) an Kartoffeln, Tomaten,
Pyricularis oryzae an Reis.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwekken; sie sollen in jedem Fall eine feine und gleichmässige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nicht ionogene und anionische Emulgatoren (z.B. Polyoxyethylen – Fettalkohol – Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,1 und 3 kg Wirkstoff oder mehr je ha.

Die Verbindungen können auch im Materialschutz u.a. zur Bekämpfung holzzerstörender Pilze, wie Coniophora puteana und Polystictus versicolor eingesetzt werden. Bei der Anwendung der Wirkstoffe im Materialschutz, z.B. als Fungizide für Anstrichfarben und Weich-Polyvinylchlorid, betragen die Aufwandmengen 0,05 bis 5 Gew.-% Wirkstoff, bezogen auf das Gesamtgewicht der zu konservierenden Farben bzw. des mikrozid auszurüstenden Polyvinylchlorids. Die Wirkstoffe können auch als fungizide wirksame Bestandteile öliger Holzschutzmittel zum Schutz von Holz gegen holzverfärbende Pilze eingesetzt werden. Die Anwendung erfolgt in der Weise, dass man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Giessen.

I. 20 Gewichtsteile der Verbindung des Beispiels 1 werden in 3 Gewichtsteilen des Natriumsalzes der Diisobutylnaphthalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.

II. 3 Gewichtsteile der Verbindung des Beispiels 2 werden mit 97 Gewichtsteilen feinteiligem Kaolin innig vermischt. Man erhält auf diese Weise ein Stäubemittel, das 3 Gew.-% des Wirkstoffs enthält.

III. 30 Gewichtsteile der Verbindungen des Beispiels 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV. 40 Gewichtsteile der Verbindung des Beispiels 3 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wässrige Dispersion. Durch Verdünnen mit Wasser erhält man eine wässrige Dispersion.

V. 20 Teile der Verbindung des Beispiels 2 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

VI. Man vermischt 90 Gewichtsteile der Verbindung des Beispiels 1 mit 10 Gewichtsteilen N-Methyl-alpha-pyrrolidon und erhält eine Lösung, die zur Anwendung in Form kleinster Tropfen geeignet ist.

VII. 20 Gewichtsteile der Verbindung des Beispiels 3 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in Wasser erhält man eine wässrige Dispersion.

VIII. 20 Gewichtsteile der Verbindung des Beispiels 2 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingiessen und feines Verteilen der Lösung in Wasser erhält man eine wässrige Dispersion.

IX. 10 Gewichtsteile der Verbindung des Beispiels 3, 20 Gewichtsteile Polyoxyethylensorbitan-monolaurat, 20 Gewichtsteile Methanol und 50 Gewichtsteile Wasser werden zu einer Lösung verrührt, die 10 Gew.-% des Wirkstoffs enthält. Durch Hinzufügen von weiterem Wasser können stärker verdünnte Lösungen hergestellt werden.

Die Mittel können in diesen Aufwendungsformen auch zusammen mit anderen Wirkstoffen

vorliegen, wie z.B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrösserung des Wirkungsspektrums. Bei einer Anzahl solcher Fungizidmischungen treten auch synergistische Effekte auf, d.h. die Wirksamkeit des Kombinationsproduktes ist grösser als die addierten Wirksamkeiten der Einzelkomponenten.

Fungizide, die mit den erfindungsgemässen Verbindungen kombiniert werden können, sind beispielsweise:

Schwefel,

Dithiocarbamate und deren Derivate, wie

Ferridimethyldithiocarbamat

Zinkdimethyldithiocarbamat

Zinkethylenbisdithiocarbamat

Manganethylenbisdithiocarbamat

Mangan-Zink-ethylendiamin-bis-dithiocarbamat

Tetramethylthiuramidsulfide

Ammoniak-Komplex von Zink-(N,N'-ethylen-bis-dithiocarbamat)

Ammoniak-Komplex von Zink-(N,N'-propylen-bis-dithiocarbamat)

Zink-(n,N'-propylen-bis-dithiocarbamat)

N,N'-Polypropylen-bis-(thiocarbamoyl)-disulfid

Nitroderivate, wie

Dinitro-(1-methylheptyl)-phenylcrotonat

2-sec.-Butyl-4,6-dinitrophenyl-3,3-dimethylacrylat

2-sec.-Butyl-4,6-dinitrophenyl-isopropylcarbonat

5-Nitro-isophthalsäure-di-isopropylester,

heterocyclische Verbindungen, wie

2-Heptadecyl-2-imidazolin-acetat

2,4-Dichlor-6-(o-chloranilino)-s-triazin

O,O-Diethyl-phthalimidophosphonothioat

5-Amino-1-(bis-(dimethylamino)-phosphinyl)-3-phenyl-1,2,4-triazol

2,3-Dicyano-1,4-dithiaanthrachinon

2-Thio-1,3-dithio-(4,5,6)-chinoxalin

1-(Butylcarbamoyl)-2-benzimidazol-carbaminsäuremethylester

2-Methoxycarbonylamino-benzimidazol

2-(Furyl-(2))-benzimidazol

2-(Thiazolyl-(4))-benzimidazol

N-(1,1,2,2-Tetrachlorethylthio)-tetrahydrophthalimid

N-Trichlormethylthio-tetrahydrophthalimid

N-Trichlormethyl-phthalimid

N-Dichlorfluormethylthio-N',N'-dimethyl-N-phenyl-schwefelsäurediamid

5-Ethoxy-3-trichlormethyl-1,2,3-thiadiazol

2-Rhodanmethylthiobenzthiazol

1,4-Dichlor-2,5-dimethoxybenzol

4-(2-Chlorphenylhydrazono)-3-methyl-5-isoxazolon

Pyridin-2-thio-1-oxid

8-Hydroxychinolin bzw. dessen Kupfersalz

2,3-Dihydro-5-carboxanilido-6-methyl-1,4-oxathiin

2,3-Dihydro-5-carboxanilido-6-methyl-1,4-oxathiin-4,4-dioxid

2-Methyl-5,6-dihydro-4-H-pyran-3-carbonsäure-anilid

2-Methyl-furan-3-carbonsäureanilid

2,5-Dimethyl-furan-3-carbonsäureanilid

2,4,5-Trimethyl-furan-3-carbonsäureanilid

2,5-Dimethyl-furan-3-carbonsäurecyclohexylamid

N-Cyclohexyl-N-methoxy-2,5-dimethyl-furan-3-carbonsäureamid

2-Methyl-benzoesäure-anilid

2-Jod-benzoesäure-anilid

N-Formyl-N-morpholin-2,2,2-trichlorethylacetal

Piperazin-1,4-diylbis(1-(2,2,2-trichlor-ethyl)-formamid

1-(3,4-Dichloranilino)-1-formylamino-2,2,2-trichlorethan

2,6-Dimethyl-N-tridecyl-morpholin bzw. dessen Salze

2,6-Dimethyl-N-cyclododecyl-morpholin bzw. dessen Salze

N-[3-(p-tert.-Butylphenyl)-2-methylpropyl]-cis-2,6-dimethylmorpholin

N-[3-(p-tert.-Butylphenyl)-2-methylpropyl]-piperidin

1-[2-(3,4-Dichlorphenyl)-4-ethyl-1,3-dioxolan-2-yl-ethyl]-1-H-1,2,4-triazol

1-[2-(2,4-Dichlorphenyl)-4-n-propyl-1,3-dioxolan-2-yl-ethyl]-1-H-1,2,4-triazol

N-(n-Propyl)-N-(2,4,6-trichlorphenoxyethyl)-N'-imidazol-yl-harnstoff

1-(4-Chlorphenoxy)-3,3-dimethyl-1-(1H-1,2,4-triazol-1-yl)-2-butanon

1-(4-Chlorphenoxy)-3,3-dimethyl-1-(1H-1,2,4-triazol-1-yl)-2-butanol

α-(2-Chlorphenyl)-(4-chlorphenyl)-5-pyrimidin-methanol

5-Butyl-2-dimethylamino-4-hydroxy-6-methyl-pyrimidin

Bis-(p-chlorphenyl)-3-pyridinmethanol

1,2-Bis-(3-ethoxycarbonyl-2-thioureido)-benzol

1,2-Bis-(3-methoxycarbonyl-2-thioureido)-benzol

sowie verschiedene Fungizide, wie

Dodecylguanidinacetat

3-(3-(3,5-Dimethyl-2-oxycyclohexyl)-1-hydroxyethyl)-glutarimid

Hexachlorbenzol

DL-Methyl-N-(2,6-dimethyl-phenyl)-N-furoyl(2)-alaninat,

DL-N-(2,6-Dimethyl-phenyl)-N-(2'-methoxyacetyl)-alaninmethylester,

N-(2,6-Dimethylphenyl)-N-chloracetyl-D,L-2-aminobutyrolacton,

DL-N-(2,6-Dimethylphenyl)-N-(phenylacetyl)-alaninmethylester

5-Methyl-5-vinyl-3-(3,5-dichlorphenyl)-2,4-dioxo-1,3-oxazolidin

3-(3,5-Dichlorphenyl)-5-methyl-5-methoxymethyl-1,3-oxazolidin-2,4-dion

3-(3,5-Dichlorphenyl)-1-isopropylcarbamoylhydantoin

N-(3,5-Dichlorphenyl)-1,2-dimethylcyclopropan-1,2-dicarbonsäureimid

2-Cyano-N- (ethylaminocarbonyl) -2-methoxyimino)-acetamid.

Die folgenden Versuche erläutern die biologische Wirkung der erfindungsgemässen Verbindungen. Für die Versuche wurden die folgenden bekannten Wirkstoffe zum Vergleich verwendet:
1-(2,4-Dichlorphenyl)-3- (1,2,4-triazolyl-1-)-4,4-dimethyl-pentanon-1 (A)
(DE-OS 27–39 352)
1-(2,4-Dichlorphenyl)-3- (1,2,4-triazolyl-1-)-4,4-dimethyl-pentanol-1 (B)
(DE-OS 27 38 725)
1-(2,4-Dichlorphenyl)-3- (1,2,4-triazolyl-1)-4,4-dimethyl-pentan-1-on-oxim (C)
(DE-OS 28 42 801).

Versuch 1

Wirksamkeit gegen Weizenmehltau

Blätter von in Töpfen gewachsenen Weizenkeimlingen der Sorte «Jubilar» werden mit wässriger Spritzbrühe, die 80% Wirkstoff und 20% Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschliessend im Gewächshaus bei Temperaturen zwischen 20 und 22 °C und 75 bis 80% relativer Luftfeuchtigkeit aufgestellt. Nach 7 Tagen wird das Ausmass der Mehltauentwicklung ermittelt.

Das Ergebnis des Versuches zeigt, dass beispielsweise die Verbindungen Nr. 1, 2, 3, 14, 15, 16, 19, 21, 31, 38, 48, 49, 51, 57, 60, 61, 62, 65, 66 und 85 bei der Anwendung als 0,05; 0,025; 0,006 oder 0,0015%ige (Gew.-%) Spritzbrühe eine bessere fungizide Wirkung (97%) haben als die bekannten Wirkstoffe A und C (50%).

Versuch 2

Wirksamkeit gegen Weizenbraunrost

Blätter von in Töpfen gewachsenen Weizensämlingen der Sorte «Jubilar» werden mit Sporen des Braunrostes (Puccinia recondita) bestäubt. Danach werden die Töpfe für 24 Stunden bei 20 bis 22 °C in eine Kammer mit hoher Luftfeuchtigkeit (90 bis 95%) gestellt. Während dieser Zeit keimen die Sporen aus und die Keimschläuche dringen in das Blattgewebe ein. Die infizierten Pflanzen werden anschliessend mit wässrigen Spritzbrühen, die 80% Wirkstoff und 20% Emulgiermittel in der Trockensubstanz enthalten, tropfnass gespritzt. Nach dem Antrocknen des Spritzbelages werden die Versuchspflanzen im Gewächshaus bei Temperaturen zwischen 20 und 22 °C und 65 bis 70% relativer Luftfeuchte aufgestellt. Nach 8 Tagen wird das Ausmass der Rostpilzentwicklung auf den Blättern ermittelt.

Das Ergebnis des Versuches zeigt, dass beispielsweise die Verbindungen Nr. 1, 2, 3, 14, 15, 16, 31, 57, 58, 65, 67 und 77 bei der Anwendung als 0,05 oder 0,025%ige Spritzbrühe eine bessere fungizide Wirkung (97%) zeigen als die bekannten Wirkstoffe A, B und C (0%).

Versuch 3

Wirksamkeit gegen Gerstenmehltau

Blätter von in Töpfen gewachsenen Gerstenkeimlingen der Sorte «Asse» werden mit wässriger Spritzbrühe, die 80% Wirkstoff und 20% Emulgiermittel in der Trockensubstanz enthält, besprüht und nach 24 Stunden mit Oidien (Sporen) des Gerstenmehltaus (Erysiphe graminis f. sp. hordei) bestäubt. Die Versuchspflanzen werden anschliessend im Gewächshaus bei Temperaturen zwischen 20 und 22 °C und 75 bis 80% relativer Luftfeuchtigkeit aufgestellt. Nach 7 Tagen wird das Ausmass der Mehltauentwicklung ermittelt.

Das Ergebnis des Versuches zeigt, dass beispielsweise die Verbindungen Nr. 3, 14, 15, 16, 19, 20, 34, 38, 43, 44, 49, 51, 52, 56, 57, 60, 61, 66, 67, 77 und 85 als 0,05; 0,006 und 0,015%ige Spritzbrühe eine bessere fungizide Wirkung (97%) zeigen als die bekannten Wirkstoffe A, B und C (60%).

Versuch 4

Wirksamkeit gegen Botrytis cinerea an Paprika

Paprikasämlinge der Sorte «Neusiedler Ideal Elite» werden, nachdem sich 4 bis 5 Blätter gut entwickelt haben, mit wässrigen Suspensionen, die 80% Wirkstoff und 20% Emulgiermittel in der Trockensubstanz enthalten, tropfnass gespritzt. Nach dem Antrocknen des Spritzbelages werden die Pflanzen mit einer Konidienaufschwemmung des Pilzes Botrytis cinerea besprüht und bei 22 bis 24 °C in eine Kammer mit hoher Luftfeuchtigkeit gestellt. Nach 5 Tagen hat sich die Krankheit auf den unbehandelten Kontrollpflanzen so stark entwickelt, dass die entstandenen Blattnekrosen den überwiegenden Teil der Blätter bedecken.

Das Ergebnis des Versuches zeigt, dass beispielsweise die Verbindungen Nr. 2, 15, 19, 29, 31, 57, 58, 61, 67 und 77 bei der Anwendung als 0,05%ige Spritzbrühe eine gute fungizide Wirkung (97%) zeigen.

**Patentansprüche für die Vertragsstaaten BE, CH, DE, FR, GB, IT, LI, NL, SE**

1. alpha-Neopentyl-phenethyltriazole der Formel I

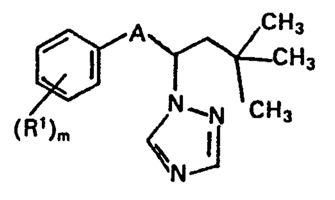

(I),

in der $R^1$ $C_1$–$C_4$-Alkyl, Phenyl, $CF_3$, $C_1$–$C_4$-Alkoxy oder Halogen, m 0, 1, 2, 3,

A C=X, CHY oder $CR^2R^3$ bedeuten, wobei X für O und $NOR^4$ und Y für $OR^5$, $OC_1$–$C_4$-Acyl, OBenzol oder $OSi(CH_3)_3$ stehen und $R^2$ OH oder $OCH_3$

$R^3$ $C_1$–$C_4$-Alkyl, Benzyl, 4-Cl-Benzyl, Phenyl, 4-$CH_3$-Phenyl, 4–Cl-Phenyl, 4–Br-Phenyl oder Vinyl,

$R^4$ und $R^5$ H, $C_1$–$C_6$-Alkyl, Allyl, Propargyl, Benzyl, 4–F-Benzyl oder 4–$CH_3$-Benzyl bedeu-

ten, sowie die pflanzenverträglichen Salze und Metallkomplexe dieser Verbindungen.

2. Fungizides Mittel enthaltend ein alpha-Neopentyl-phenethyltriazol gemäss Anspruch 1.

3. Verfahren zur Bekämpfung von Pilzen, dadurch gekennzeichnet, dass man eine fungizid wirksame Menge einer Verbindung der Formel I gemäss Anspruch 1 auf durch Pilzbefall bedrohte Materialien, Flächen, Pflanzen oder Saatgüter oder auf Pilze einwirken lässt.

4. Verfahren zur Herstellung der α-Neopentyl-phenethyltriazole gemäss Anspruch 1, dadurch gekennzeichnet, dass man ein Bromketon der Formel

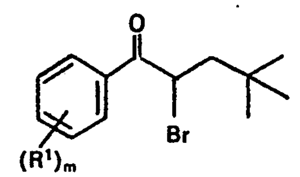

in Gegenwart eines Säurefängers und eines Lösungsmittels zunächst mit 1,2,4-Triazol umsetzt und die so erhaltene Ketonverbindung gegebenenfalls danach in üblicher Weise zu dem entsprechenden Alkohol, Ether, Oxim oder Oximether umsetzt.

5. Fungizides Mittel, enthaltend einen Trägerstoff und ein alpha-Neopentyl-phenethyl-triazol gemäss Anspruch 1.

**Patentansprüche für den Vertragsstaat AT**

1. Fungizides Mittel, enthaltend ein alpha-Neopentyl-phenethyltriazol der Formel I

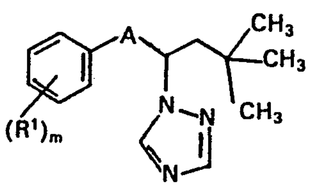 (I),

in der $R^1$ $C_1$–$C_4$-Alkyl, Phenyl, $CF_3$, $C_1$–$C_4$-Alkoxy oder Halogen, m 0, 1, 2, 3,

A C=X, CHY oder $CR^2R^3$ bedeuten, wobei X für O und $NOR^4$ und Y für $OR^5$, $OC_1$–$C_4$-Acyl, OBenzoyl oder $OSi(CH_3)_3$ stehen und $R^2$ OH oder $OCH_3$

$R^3$ $C_1$–$C_4$-Alkyl, Benzyl, 4–Cl-Benzyl, Phenyl, 4–$CH_3$-Phenyl, 4–Cl-Phenyl, 4–Br-Phenyl oder Vinyl,

$R^4$ und $R^5$ H, $C_1$–$C_6$-Alkyl, Allyl, Propargyl, Benzyl, 4–F-Benzyl oder 4–$CH_3$-Benzyl bedeuten, sowie die pflanzenverträglichen Salze und Metallkomplexe dieser Verbindungen.

2. Fungizid enthaltend eine Verbindung der Formel I gemäss Anspruch 1 und einen festen oder flüssigen Trägerstoff.

3. Verfahren zur Bekämpfung von Pilzen, dadurch gekennzeichnet, dass man eine fungizid wirksame Menge einer Verbindung der Formel I gemäss Anspruch 1 auf durch Pilzbefall bedrohte Materialien, Flächen, Pflanzen oder Saatgüter oder auf Pilze einwirken lässt.

4. Verfahren zur Herstellung der α-Neopentyl-phenethyltriazole gemäss Anspruch 1, dadurch gekennzeichnet, dass man ein Bromketon der Formel

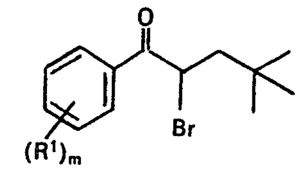

in Gegenwart eines Säurefängers und eines Lösungsmittels zunächst mit 1,2,4-Triazol umsetzt und die so erhaltene Ketonverbindung gegebenenfalls danach in üblicher Weise zu dem entsprechenden Alkohol, Ether, Oxim oder Oximether umsetzt.

**Revendications pour les Etats contractants: BE, CH, DE, FR, GB, IT, LI, NL, SE**

1. alpha-néopentyl-phénéthyltriazoles de formule I

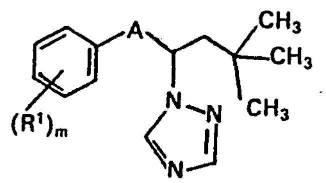 (I),

dans laquelle

$R^1$ représente alkyle en $C_1$–$C_4$, phényle, $CF_3$, alcoxy en $C_1$–$C_4$ ou halogène,

m représente 0, 1, 2, 3,

A représente C=X, CHY ou $CR^2R^3$, X étant mis pour O et $NOR^4$ et Y pour $OR^5$, O-acyle en $C_1$–$C_4$, O-benzoyle ou $OSi(CH_3)_3$, et

$R^2$ représente OH ou $OCH_3$

$R^3$, alkyle en $C_1$–$C_4$, benzyle, 4-Cl-benzyle, phényle, 4–$CH_3$-phényle, 4–Cl-phényle, 4–Br-phényle ou vinyle,

$R^4$ et $R^5$ représentent H, alkyle en $C_1$–$C_6$, allyle, propargyle, benzyle, 4–F-benzyle, ou 4–$CH_3$-benzyle, ainsi que les sels acceptables pour les plantes et les complexes métalliques de ces composés.

2. Agent fongicide contenant un alpha-néopentyl-phénéthyltriazole selon la revendication 1.

3. Procédé pour lutter contre les champignons, caractérisé par le fait que l'on fait agir une quantité efficace, au point de vue fingicide, d'un composé de formule I selon la revendication 1, sur les matériaux, surfaces, plantes ou semences menacés par les champignons ou sur les champignons eux-mêmes.

4. Procédé de préparation des α-néopentyl-phénéthyltriazoles selon la revendication 1, caractérisé par le fait que l'on fait réagir une cétone bromée de formule

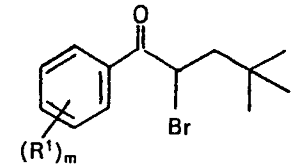

en présence d'un fixateur d'acide et d'un solvant, d'abord avec du 1,2,4-triazole et l'on transforme, éventuellement après, le composé cétonique ainsi obtenu en alcool, éther, oxime ou oximéther correspondant.

5. Agent fongicide, contenant un véhicule et un α-néopentyl-phénéthyl-triazole selon la revendication 1.

**Revendications pour l'Etat contracant: AT**

1. Agent fongicide contenant un alpha-néopentyl-phénéthyl-triazole de formule I

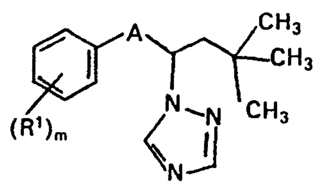

(I),

dans laquelle

$R^1$ représente alkyle en $C_1$–$C_4$, phényle, $CF_3$, alcoxy en $C_1$–$C_4$ ou halogène,

m représente 0, 1, 2, 3,

A représente C=X, CHY ou $CR^2R^3$, X étant mis pour 0 es $NOR^4$ et Y pour $OR^5$, O-acyle en $C_1$–$C_4$, O-benzoyle ou $OSi(CH_3)_3$, et

$R^2$ représente OH ou $OCH_3$

$R^3$, alkyle en $C_1$–$C_4$, benzyle, 4–Cl-benzyle, phényle, 4–$CH_3$-phényle, 4–Cl-phényle, 4–Br-phényle ou vinyle,

$R^4$ et $R^5$ représentent H, alkyle en $C_1$–$C_6$, allyle, propargyle, benzyle, 4–F-benzyle, ou 4–$CH_3$-benzyle, ainsi que les sels acceptables pour les plantes et les complexes métalliques de ces composés.

2. Fongicide contenant un composé de formule I selon la revendication 1 et un véhicule solide ou liquide.

3. Procédé pour lutter contre les champignons, caractérisé par le fait que l'on fait agir une quantité efficace, au point de vue fingicide, d'un composé de formule I selon la revendication 1, sur les matériaux, surfaces, plantes ou semences menacés par les champignons ou sur les champignons eux-mêmes.

4. Procédé de préparation des α-néopentyl-phénéthyl-triazole selon la revendication 1, caractérisé par le fait que l'on fait réagir une cétone bromée de formule

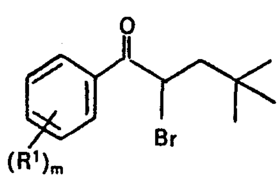

en présence d'un fixateur d'acide et d'un solvant, d'abord avec du 1,2,4-triazole et l'on transforme, éventuellement après, le composé cétonique ainsi obtenu en alcool, éther, oxime ou oximéther correspondant.

**Claims for the Contracting states: BE, CH, DE, FR, GB, IT, LI, NL, SE**

1. An alpha-neopentyl-phenethyltriazole of the formula I

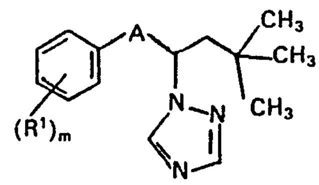

(I),

where

$R^1$ is $C_1$–$C_4$-alkyl, phenyl, $CF_3$, $C_1$–$C_4$-alkoxy or halogen, m is 0, 1, 2 or 3,

A is C=X, CHY or $CR^2R^3$, where X is 0 or $NOR^4$, and Y is $OR^5$, O–$C_1$–$C_4$-acyl, O-benzoyl or $OSi(CH_3)_3$, and

$R^2$ is OH or $OCH_3$,

$R^3$ is $C_1$–$C_4$-alkyl, benzyl, 4–Cl-benzyl, phenyl, 4–$CH_3$-phenyl, 4–Cl-phenyl, 4–Br-phenyl or vinyl, and

$R^4$ and $R^5$ are each H, $C_1$–$C_6$-alkyl, allyl, propargyl, benzyl, 4–F-benzyl or 4–$CH_3$-benzyl, and a plant-tolerated salz or metal complex of this compound.

2. A fungicidal agent containing an alpha-neopentyl-phenethyltriazole as claimed in claim 1.

3. A process for combating fungi, wherein a fungicidally effective amount of a compound of the formula I as claimed in claim 1 is allowed to act on materials, areas, plants or seed threatened by fungus attack, or on the fungi themselves.

4. A process for the preparation of an α-neopentyl-phenethyltriazole as claimed in claim 1, wherein first of all a bromoketone of the formula

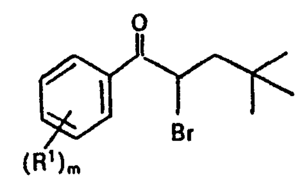

is reacted with 1,2,4-traizole in the presence of an acid acceptor and a solvent, and then, if desired, the keto compound thus obtained is reacted in a conventional manner to form the corresponding alcohol, ether, oxime or oxime ether.

5. A fungicidal agent containing a carrier and an alphaneopentyl-phenethyltriazole as claimed in claim 1.

**Claims for the Contracting state: AT**

1. A fungicidal agent containing an alpha-neopentyl-phenethyltriazole of the formula I

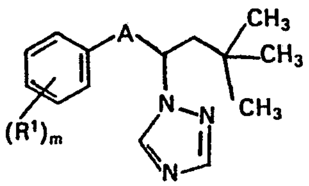

(I),

where

$R^1$ is $C_1$–$C_4$-Alkyl, phenyl, $CF_3$, $C_1$–$C_4$-alkoxy or halogen, m is 0, 1, 2 or 3,

A is C=X, CHY or $CR^2R^3$, where X is 0 or $NOR^4$, and Y is $OR^5$, O–$C_1$–$C_4$-acyl, O-benzoyl or $OSi(CH_3)_3$, and

$R^2$ is OH or $OCH_3$,

$R^3$ is $C_1$–$C_4$-alkyl, benzyl, 4–Cl-benzyl, phenyl, 4–$CH_3$-phenyl, 4–Cl-phenyl, 4–Br-phenyl or vinyl, and

$R^4$ and $R^5$ are each H, $C_1$–$C_6$-alkyl, allyl, propargyl, benzyl, 4–F-benzyl or 4–$CH_3$-benzyl, or a plant-tolerated salt or metal complex of this compound.

2. A fungicide containing a compound of the formula I as claimed in claim 1 and a solid or liquid carrier.

3. A process for combating fungi, wherein a fungicidally effective amount of a compound of the formula I as claimed in claim 1 is allowed to act on materials, areas, plants or seed threatened by fungus attack, or on the fungi themselves.

4. A process for the preparation of an α-neopentyl-phenethyltriazole as defined in claim 1, wherein first of all a bromoketone of the formula

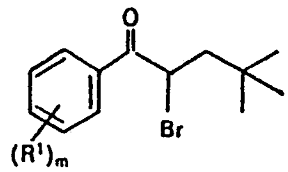

is reacted with 1,2,4-triazole in the presence of an acid acceptor and a solvent, and then, if desired, the keto compound thus obtained is reacted in a conventional manner to form the corresponding alcohol, ether, oxime or oxime ether.